(19) Europäisches Patentamt
European Patent Office
Office européen des brevets

(11) Veröffentlichungsnummer: **0 155 565**
**B1**

(12) **EUROPÄISCHE PATENTSCHRIFT**

(45) Veröffentlichungstag der Patentschrift: **25.04.90**

(51) Int. Cl.$^5$: **G 01 N 33/86,** C 12 Q 1/56

(21) Anmeldenummer: **85102281.4**

(22) Anmeldetag: **28.02.85**

(54) **Testsatz zur Bestimmung der aktivierten partiellen Thromboplastinzeit (PTT) mit erhöhter Heparinempfindlichkeit.**

(30) Priorität: **28.02.84 DE 3407280**

(43) Veröffentlichungstag der Anmeldung:
**25.09.85 Patentblatt 85/39**

(45) Bekanntmachung des Hinweises auf die Patenterteilung:
**25.04.90 Patentblatt 90/17**

(84) Benannte Vertragsstaaten:
**AT BE CH DE FR GB IT LI LU NL SE**

(56) Entgegenhaltungen:
**EP-A-0 049 877**
**EP-A-0 107 383**

(73) Patentinhaber: **AKZO N.V.**
**Velperweg 76**
**NL-6824 BM Arnhem (NL)**

(72) Erfinder: **Witt, Peter, Dr.**
**Vogelwäldeleweg 6**
**D-7844 Neuenburg (DE)**

(74) Vertreter: **Hermans, Franciscus G.M. et al**
**Patent Department AKZO N.V. Pharma Division**
**P.O. Box 20**
**NL-5340 BH Oss (NL)**

**EP 0 155 565 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft einen Testsatz zur Durchführung eines Screening-Testes (Suchtestes) für das endogene Gerinnungssystem.

In vivo beginnt der endogene Gerinnungsablauf mit zwei nebeneinander ablaufenden Prozessen:

Über die visköse Metamorphose der Thrombozyten wird der Plättchenfaktor 3 freigesetzt. Gleichzeitig kommt es zur Aktivierung des Kontaktfaktors XII durch unphysiologische Oberflächen (Kollagen, Zellfragmente), die durch die Gefäßschädigung entstanden sind. Die Faktoren XIIa, XI, Plättchenfaktor 3 und freie Calciumionen bilden nun den Faktor-IX-Aktivator. Dieser Faktor-IX-Aktivator aktiviert Faktor IX zu Faktor IXa. Faktor IXa bewirkt zusammen mit Faktor VIII und Calciumionen die Bildung eines Faktor-X-Aktivators. Dieser Aktivator besteht also aus Faktor IXa, VIII, Plättenchenfaktor 3 und Calciumionen. Er aktiviert den Faktor X zu Faktor Xa. Dieser stellt zusammen mit Faktor V, Plättchenfaktor 3 und Calciumionen den sogenannten Prothrombin-Aktivator dar, der den Faktor II (Prothrombin) zu Thrombin umwandelt.

Thrombin spaltet zwei Peptidketten vom Fibrinogen-Molekül ab, wodurch Fibrin gebildet wird. Durch End-zu-End- und Seit- zu-Seit-Anlagerungen dieser Fibrin-Monomeren entstehen die polymeren Fibrillen, das sogenannte $Fibrin_s$. Diese Fibrillen werden unter Einwirkung des aktivierten Faktors XIII durch Quervernetzung zum $Fibrin_i$ weiter polymerisiert.

In vitro wird der Ablauf des endogenen Gerinnungssystems durch ein Reagenz ausgelöst, das feinstgemahlene Partikel zur Simulation einer Fremdoberfläche sowie ein Phospholipid zur Simulation des Plättchenfaktors 3 enthält.

Dieser Test wird als Bestimmung der aktivierten partiellen Thromboplastinzeit, synomym als PTT-Bestimmungf, bezeichnet. Die PTT-Bestimmung wird besonders als präoperativer Screening-Test durchgeführt und spricht auf Störungen der Gerinnungsfaktoren I, II, V, VIII, IX, X, XI und XII an.

Auch im Zusammenhang mit der Kontrolle einer oralen Antikoagulantien-Therapie, bei der die Aktivität der Faktoren II, VII, IX und X vermindert wird, wird die PTT-Bestimmung durchgeführt, da bei der Kontrolle der oralen Antikoagulantien-Therapie durch einen anderen Test (den sogenannten Quick-Test) der Faktor IX nicht erfaßt und damit nicht kontrolliert wird.

Des weiteren erfolgt die PTT-Bestimmung zur Steuerung der therapeutischen Maßnahmen bei einer bekannten Gerinnungsstörung (z.B. Hämophilie A oder B) sowie zur Überwachung einer Heparin-Therapie.

Daraus ergibt sich, daß ein Reagenz zur Durchführung der PTT-Bestimmung gegenüber den Faktoren des endogenen Gerinnungssystems und gegenüber Heparin empfindlich reagieren muß.

Ebenfalls von entscheidender Bedeutung ist eine gute Stabilität des rekonstituierten Reagenzes bei 37°C, Raumtemperatur und Aufbewahrung im Kühlschrank.

Durchführung des Tests:

Die PTT-Bestimmung wird nach folgendem Schema durchgeführt:

| | |
|---|---|
| Plättchenfaktor-Reagenz (37°C) | 0,1 ml |
| Patientenplasma (Raumtemperatur) | 0,1 ml |
| Mischen und genau 2 Minuten bei 37°C | |
| inkubieren 0,025 M $CaCl_2$-Lösung (37°C) | 0,1 ml |
| Zeit von der Calciumchloridzugabe bis zum | |
| Gerinnungseintritt (=PTT) ermitteln | |

Wie das Schema zeigt, erfolgt die Bestimmung der PTT in zwei Stufen:

In der ersten Stufe wird der kontaktsensible Faktor XII im Plasma durch das zugesetzte Plättchenfaktor-Reagenz aktiviert und leitet den Gerinnungsablauf im endogenen System ein. Eine ausreichende Aktivierung der an der ersten Gerinnungsphase beteiligten Faktoren wird bereits innerhalb einer Inkubationszeit von 2 Minuten erreicht.

In der zweiten Stufe wird nach Ablauf der Inkubationszeit dem Ansatz Calciumchlorid-Lösung zugesetzt. Der sich bildende Prothrombinumwandlungsfaktor (Komplex aus Faktor Xa, Faktor V, Phospholipid und Calciumionen) bewirkt die Bildung von Thrombin, wodurch Fibrinogen in Fibrin umgewandelt wird. Die PTT wird vom Zusatz der Calciumionen bis zum Gerinnungseintritt gemessen.

Das Ergebnis der Messung wird in Sekunden angegeben.

Es ist aber auch möglich, das Ergebnis als Quotient anzugeben, indem man die PTT des Patienten durch die PTT eines Normalplasmapools teilt.

Nimmt man anstelle eines Patientenplasmas z.B. ein Faktor VIII- oder Faktor IX-Mangelplasma und teilt die Ergebnisse durch die PTT eines Normalplasmas, erhält man ebenfalls einen Quotienten, der um so höher ist, je empfindlicher das eingesetzte Reagenz auf einen Mangel des entsprechenden Faktors reagiert.

Zur Durchführung der PTT-Bestimmung gibt es bereits einen Testsatz, mit dem das benötigte Reagenz in Form eines Lyophilisats zur Rekonstitution bereitgestellt wird.

Nachteilig an dem handelsüblichen Testsatz zur PTT-Bestimmung ist, daß die Heparin-Empfindlichkeit noch erheblich zu wünschen übrig läßt, so daß er insbesondere nicht zur Kontrolle der Haparin-Therapie eingesetzt werden kann.

Der Erfindung lag die Aufgabe zugrunde, die Heparin-Empfindlichkeit, insbesondere die Empfindlichkeit gegenüber niedermolekularen Heparinfraktionen des bekannten Testsatzes zur PTT-Bestimmung soweit zu erhöhen, daß er auch zur Kontrolle der Heparin-Therapie einsetzbar ist.

Gegenstand der Erfindung ist somit ein Testsatz zur PTT-Bestimmung mit einem Plättchenfaktor-Reagenz und gegebenenfalls feinstgemahlenen Partikeln, welcher dadurch gekennzeichnet ist, daß er zusätzlich mindestens eine Verbindung der Formeln

$$R—O—SO_3X \text{ oder } R—SO_3X$$

worin

R für einen gegebenenfalls substituierten, ein- oder mehrfach ungesättigten, gerad- oder verzweigtkettigen aliphatischen oder cycloaliphatischen Kohlenwasserstoffrest oder einen gegebenenfalls substituierten aromatischen Rest steht und

X ein Wasserstoffatom oder ein Kation darstellt,

in einer solchen Menge enthält, dass 1 l der rekonstituierten Lösung 0,05 bis 100 mmol, vorzugsweise 0,2 mmol bis 20 mmol enthält.

Der durch R wiedergegebene Rest kann für einen aliphatischen oder cycloaliphatischen Rest mit mindestens 4 Kohlenstoffatomen, z.B. eine Butyl-, Isobutyl-, tert.-Butyl-, Pentyl-, Pentenyl-, Pentadienyl-, Cyclohexyl-, Cyclohexenyl-, Octyl-, Nonyl-, Decyl- oder Dodecylrest oder einen aromatischen Rest, z.B. einen Phenyl-, Naphthyl-, Penanthryl-, Anthryl-, Pyridyl-, Furyl-, Benzofuryl-, Isobenzofuryl-, Thienyl-, Chinolyl-, Isochinolyl-, Phenanthrolinyl-, Pyrrolyl-, Pyrimidinylrest, stehen.

In diesen Resten R kann (können) ein oder mehrere Wasserstoffatom(e) beispielsweise durch Methyl-, Ethyl-, Propyl-, Octyl-, Decyl-, Pentyl-, Chorpentyl-, Isopropyl-, Pentenyl-, Hydroxy-, Amino-, Nitro-Gruppen oder Jod-, Chlor-, Fluor- oder Bromatome substituiert sein.

Beispiele für Verbindungen der Formel $R—O—SO_3X$ sind: Natriumlaurylsulfat, 3,5-Dimethylcyclohexansulfat-Na-Salz, Isononansulfat-Na-Salz, 5,6-Dimethylheptansulfat-Na-Salz, 10-Undecensulfat-Na-Salz, Undecansulfat-Na-Salz.

Beispiele für Verbindungen der Formel $R—SO_3X$ sind: Dodecylbenzolsulfonsäure-Na-Salz, Pentansulfonsäure-Na-Salz, Naphthalin-4-sulfonsäure-Na-Salz, Naphthalin-2,6-disulfonsäure-Na-Salz, 1-Naphtol-2-sulfonsäure-Na-Salz, 4-Vinyl-benzolsulfonsäure-Na-Salz, 1-Octansulfonsäure-Na-Salz.

Die genannten Zusätze der Formeln $R—O—SO_2X$ und $R—SO_3X$ können in den rekonstituierten Testlösungen in Konzentrationen von 0,05 mmol/l bis 100 mmol/l, vorzugsweise zwischen 0,2 mmol/l und 20 mmol/l, vorliegen.

Die folgenden Beispiele sollen die Erfindung näher veranschaulichen.

## Beispiele

In den folgenden Beispielen wird die unterschiedliche Heparinempfindlichkeit bei Auflösung des PTT-Reagenzes in 3 ml destilliertem Wasser bzw. in der gleichen Menge einer wässrigen Lösung von Pentansulfonsäure-Na-Salz, Dodecylbenzolsulfonsäure-Na-Salz bzw. Natriumlaurylsulfat ermittelt.

In Versuch 1 werden unfraktioniertes Heparin, in den Versuchen 2 und 3 fraktionierte Heparine mit relativ niedrigen, mittleren Molekulargewichten eingesetzt. Der Start der Bestimmung erfolgt durch die $CaCl_2$-Zugabe.

Aus den in den Fig. 1 bis 3 graphisch dargestellten Ergebnissen der Versuche 1 bis 3 geht hervor, daß unabhängig von den verwendeten Heparinfraktionen die Heparinempfindlichkeit durch Zusatz der genannten Zusätze erheblich gesteigert wird.

1. Die verwendeten Plasmen wurden mit dem üblicherweise eingesetzten unfraktionierten Heparin aus Schweine Intestinal Mucosa heparinisiert. Das Heparin in den Plasmen wurde mit einem handelsüblichen Testsatz bestimmt und in USP-Einheiten/ml Plasma angegeben. In Fig. 1 ist die jeweilige Gerinnungszeit in Sekunden(s) gegen die Heparinkozentration in USP-Einheiten/ml Plasma aufgetragen.

2. Die verwendeten Plasmen wurden mit niedermolekularem Heparin, und zwar einem handelsüblichen Mucopolysaccharid eines mittleren Molukargewichts von 6000 Daltons heparinisiert. In Fig. 2 ist die jeweilige Gerinnungszeit in Sekunden(s) gegen die Heparinkonzentration in USP-Einheiten/ml Plasma aufgetragen.

3. Die verwendeten Plasmen wurden mit einem handelsüblichen niedermolekularen Heparin eines mittleren Molekulargewichts von 4040 Daltons heparinisiert. In Fig. 3 ist die jeweilige Gerinnungszeit in Sekunden(s) gegen die Heparinkonzentration in USP-Einheiten/ml Plasma aufgetragen.

**Patentansprüche für die Vertragsstaaten: BE CH DE FR GB IT LI LU NL SE**

1. Testsatz zur PTT-Bestimmung mit einem Plättchenfaktor-Reagenz, dadurch gekennzeichnet, daß er zusätzlich mindestens eine Verbindung der Formeln

$$R—O—SO_3X \text{ oder } R—SO_3X$$

worin

R für einen gegebenenfalls substituierten, ein- oder mehrfach ungesättigten, gerad- oder verzweigtkettigen aliphatischen oder cycloalphatischen Kohlenwasserstoffrest oder einen gegebenenfalls substituierten aromatischen Rest steht und

X ein Wasserstoffatom oder ein Kation darstellt,

in einer solchen Menge enthält, daß 1 l der rekonstituierten Lösung 0,05 bis 100 mmol davon enthält.

2. Testsatz nach Anspruch 1, dadurch gekennzeichnet, daß er die zusätzliche(n) Verbindung(en) in einer solchen Menge enthält, daß 1 l der rekonstituierten Lösung 0,2 bis 20 mmol davon enthält.

3. Testsatz nach Anspruch 1, dadurch gekennzeichnet, daß er Dodezylbenzolsulfonsäure-Na-Salz und/ oder Natriumlaurylsulfat enthält.

4. Testsatz nach einem oder mehreren der vorhergehenden Ansprüche, dadurch gekennzeichnet, daß er die zusätzliche(n) Verbindung(en) in Form einer Rekonstitutionslösung enthält.

**Patentansprüche für den Vertragsstaat: AT**

1. Testsatz zur PTT-Bestimmung mit einem Plättchenfaktor-Reagenz, dadurch gekennzeichnet, daß er zusätzlich mindestens eine Verbindung der Formeln

$$R—O—SO_3X \text{ oder } R—SO_3X$$

worin

R für einen gegebenenfalls substituierten, ein- oder mehrfach ungesättigten, gerad- oder verzweigtkettigen aliphatischen oder cycloalphatischen Kohlenwasserstoffrest oder einen gegebenenfalls substituierten aromatischen Rest steht und

X ein Wasserstoffatom oder ein Kation darstellt,

in einer solchen Menge enthält, daß 1 l der rekonstituierten Lösung 0,05 bis 100 mmol davon enthält.

2. Testsatz nach Anspruch 1, dadurch gekennzeichnet, daß er die zusätzliche(n) Verbindung(en) in einer solchen Menge enthält, daß 1 l der rekonstituierten Lösung 0,2 bis 20 mmol davon enthält.

3. Testsatz nach Anspruch 1, dadurch gekennzeichnet, daß er Dodezylbenzolsulfonsäure-Na-Salz und/ oder Natriumlaurylsulfat enthält.

4. Testsatz nach einem oder mehreren der vorhergehenden Ansprüche, dadurch gekennzeichnet, daß er die zusätzliche(n) Verbindung(en) in Form einer Rekonstitutionslösung enthält.

5. Verfahren zur PTT-Bestimmung mit einem Plättchenfaktor-Reagenz, dadurch gekennzeichnet, daß man dem Reagenz zusätzlich mindestens eine Verbindung der Formeln

$$R—O—SO_3X \text{ oder } R—SO_3X$$

worin

R für einen gegebenenfalls substituierten, ein- oder mehrfach ungesättigten, gerad- oder verzweigtkettigen aliphatischen oder cycloalphatischen Kohlenwasserstoffrest oder einen gegebenenfalls substituierten aromatischen Rest steht und

X ein Wasserstoffatom oder ein Kation darstellt,

in einer solchen Menge enthält, dass 1 l der rekonstituierten Lösung 0,05 bis 100 mmol davon enthält, und die Bestimmung mit dem Reagentiengemisch in an sich bekannter Weise durchführt.

6. Verfahren nach Anspruch 5, dadurch gekennzeichnet, dass man die zusätzlich(n) Verbindung(en) in einer solchen Menge zugibt, dass 1 l der rekonstituierten Lösung 0,2 bis 20 mmol davon enthält.

7. Verfahren nach Anspruch 5, dadurch gekennzeichnet, dass als Hilfsmittel zusätzlich Dodezylbenzolsulfonsäure-Na-Salz und/oder Natriumlaurylsulfat zugegeben wird.

8. Verfahren nach einem oder mehreren der Ansprüche 5—7, dadurch gekennzeichnet, dass man die zusätzliche(n) Verbindung(en) in eine Rekonstitutionslösung einbringt.

**Revendications pour les Etats contractants: BE CH DE FR GB IT LI LU NL SE**

1. Nécessaire pour la détermination du PTT comprenant un réactif du facteur plaquettaire, caractérisé en ce qu'il contient de surcroît au moins un composé de formule

$$R—O—SO_3X \text{ ou } R—SO_3X$$

où R représente un reste d'hydrocarbure cycloaliphatique ou aliphatique en chaîne droite ou ramifiée une ou plusieurs fois insaturé éventuellement substitué ou un reste aromatique éventuellement substitué et

X représente un atome d'hydrogène ou un cation,

en une quantité telle que l litre de la solution reconstituée en contienne 0,05 à 100 millimoles.

2. Nécessaire suivant la revendication 1, caractérisé en ce qu'il contient le ou les composés supplémentaires en une quantité telle que 1 litre de la solution reconstituée en contienne 0,2 à 20 millimoles.

3. Nécessaire suivant la revendication 1, caractérisé en ce qu'il contient du dodécylbenzènesulfonate de sodium et/ou du laurylsulfat de sodium.

4. Nécessaire suivant une ou plusieurs des revendications précédentes, caractérisé en ce qu'il contient le ou les composés supplémentaires sous la forme d'un solution à reconstituer.

**Revendications pour l'Etat contractant: AT**

1. Nécessaire pour la détermination du PTT comprenant un réactif du facteur plaquettaire, caractérisé en en ce qu'il contient de surcroît au moins un composé de formule

$$R—O—SO_3X \text{ ou } R—SO_3X$$

où R représente un reste d'hydrocarbure cycloaliphatique ou aliphatique en chaîne droite ou ramifiée une ou plusieurs fois insaturé éventuellement substitué ou un reste aromatique éventuellement substitué et

X représente un atome d'hydrogène ou un cation,

en une quantité telle que l litre de la solution reconstituée en contienne 0,05 à 100 millimoles.

2. Nécessaire suivant la revendication 1, caractérisé en ce qu'il contient le ou les composés supplémentaires en une quantité telle que 1 litre de la solution reconstituée en contienne 0,2 à 20 millimoles.

3. Nécessaire suivant la revendication 1, caractérisé en ce qu'il contient du dodécylbenzènesulfonate de sodium et/ou du laurylsulfat de sodium.

4. Nécessaire suivant une ou plusieurs des revendications précédentes, caractérisé en ce qu'il contient le ou les composés supplémentaires sous la forme d'un solution à reconstituer.

5. Procédé pour la détermination du PTT à l'aide d'un réactif du facteur plaquettaire, caractérisé en ce qu'ou ajoute au réactif en outre au moins un composé de formule

$$R—O—SO_3X \text{ ou } R—SO_3X$$

où R représente un reste d'hydrocarbure cycloaliphatique ou aliphatique en chaîne droite ou ramifiée une ou plusieurs fois insaturé éventuellement substitué ou un reste aromatique éventuellement substitué et

X représente un atome d'hydrogène ou un cation,

en une quantité telle que l litre de la solution reconstituée en contienne 0,05 à 100 millimoles, et on exécute la détermination avec le mélange des réactifs d'une façon connue.

6. Procédé suivant la revendication 5, caractérisé en ce qu'on ajoute le ou les composés supplémentaires en une quantité telle que 1 litre de la solution reconstituée en contienne 0,2 à 20 millimoles.

7. Procédé suivant la revendication 5, caractérisé en ce qu'on ajoute comme agents auxiliaires de surcroît du dodécylbenzènesulfonate de sodium et/ou du laurylsulfate de sodium.

8. Procédé suivant une ou plusieurs des revendications 5 à 7, caractérisé en ce qu'on incorpore le ou les composés supplémentaires à une solution à reconstituer.

**Claims for the Contracting States: BE CH DE FR GB IT LI LU NL SE**

1. Test kit for the PTT determination with a platelet factor reagent, characterized in that it additionally contains at least one compound of the formulae

$$R—O—SO_3X \text{ or } R—SO_3X$$

in which

R stands for an optionally substituted mono- or polyunsaturated, straight-chain or branched aliphatic or cycloaliphatic hydrocarbon radical or an optionally substituted aromatic radical, and

X represents a hydrogen atom or a cation,

is additionally added to the reagent in an amount such that 1 l of the reconstituted solution contains 0.05 to 100 mmol thereof.

2. Test kit according to Claim 1, characterized in that it contains the additional compound(s) in an amount such that 1 l of the reconstituted solution contains 0.2 to 20 mmol thereof.

3. Test kit according to Claim 1, characterized in that it contains dodecylbenzenesulphonic acid Na salt and/or sodium lauryl sulphate.

4. Test kit according to one or more of the preceding claims, characterized in that it contains the additional compound(s) in the form of a reconstitution solution.

**Claims for the Contracting State: AT**

1. Test kit for the PTT determination with a platelet factor reagent, characterized in that it additionally contains at least one compound of the formulae

$$R—O—SO_3X \text{ or } R—SO_3X$$

in which

R stands for an optionally substituted mono- or polyunsaturated, straight-chain or branched aliphatic or cycloaliphatic hydrocarbon radical or an optionally substituted aromatic radical, and

X represents a hydrogen atom or a cation,

in an amount such that 1 l of the reconstituted solution contains 0.05 to 100 mmol thereof.

2. Test kit according to Claim 1, characterized in that it contains the additional compound(s) in an amount such that 1 l of the reconstituted solution contains 0.2 to 20 mmol thereof.

3. Test kit according to Claim 1, characterized in that it contains dodecylbenzenesulphonic acid Na salt and/or sodium lauryl sulphate.

4. Test kit according to one or more of the preceding claims, characterized in that it contains the additional compound(s) in the form of a reconstitution solution.

5. Method for the PTT determination with a platelet factor reagent, characterized in that at least one compound of the formulae

$$R—O—SO_3X \text{ or } R—SO_3X$$

in which

R stands for an optionally substituted mono- or polyunsaturated, straight-chain or branched aliphatic or cycloaliphatic hydrocarbon radical or an optionally substituted aromatic radical, and

X represents a hydrogen atom or a cation,

is additionally added to the reagent in an amount such that 1 l of the reconstituted solution contains 0.05 to 100 mmol thereof and the determination is performed with the reagent mixture in a manner known per se.

6. Method according to Claim 5, characterized in that the additional compound(s) is added in an amount such that 1 l of the reconstituted solution contains 0.2 to 20 mmol thereof.

7. Method according to Claim 5, characterized in that dodecylbenzenesulphonic acid Na salt and/or sodium lauryl sulphate is additionally added as auxiliary.

8. Method according to one or more of Claims 5—7, characterized in that the additional compound(s) is introduced into a reconstitution solution.

FIG.1

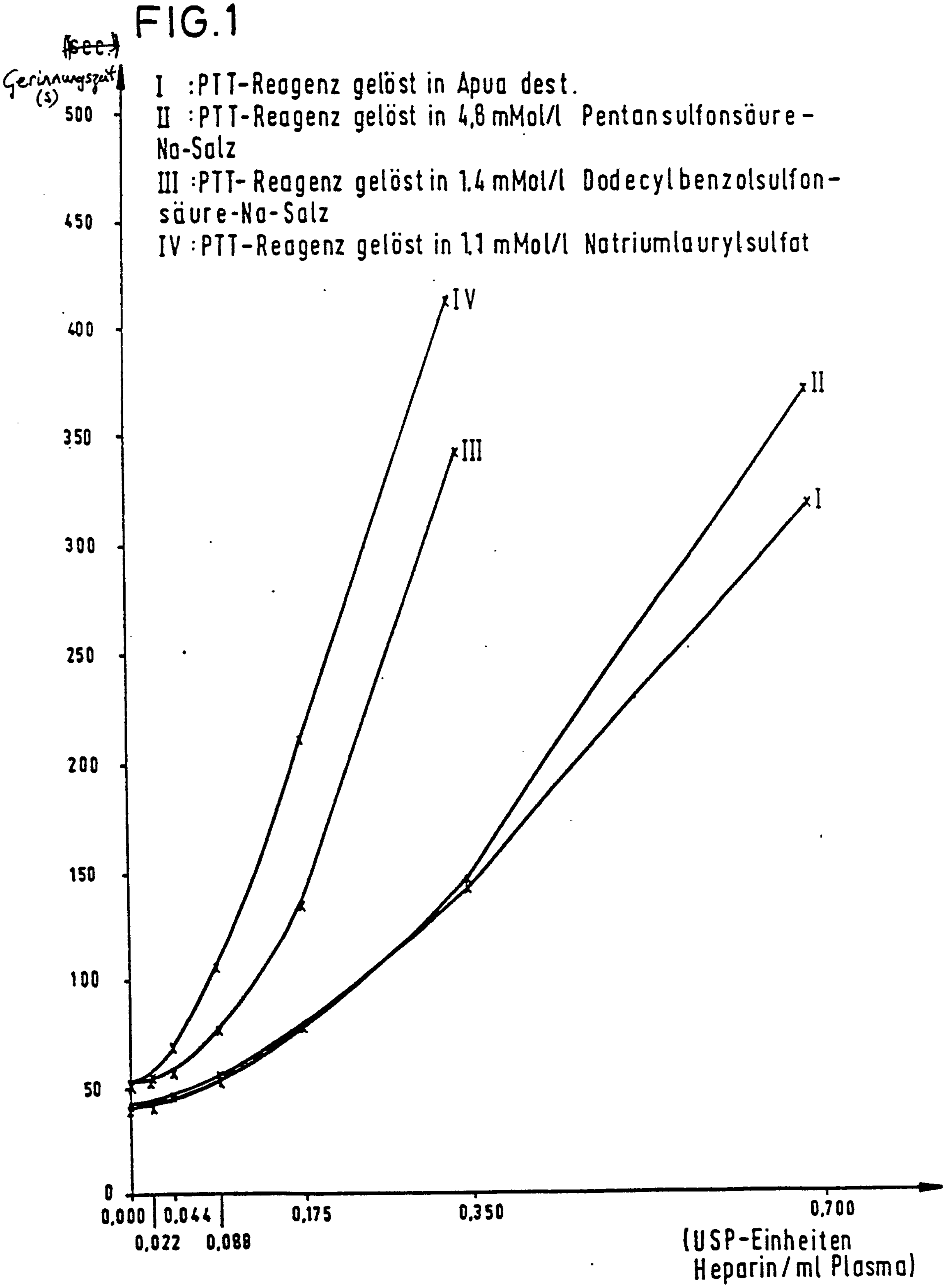
[sec.]
Gerinnungszeit (s)
500
450
400
350
300
250
200
150
100
50
0
I :PTT-Reagenz gelöst in Apua dest.
II :PTT-Reagenz gelöst in 4,8 mMol/l Pentansulfonsäure-Na-Salz
III :PTT-Reagenz gelöst in 1.4 mMol/l Dodecylbenzolsulfonsäure-Na-Salz
IV :PTT-Reagenz gelöst in 1.1 mMol/l Natriumlaurylsulfat
IV
III
II
I
0,000
0,022
0,044
0,088
0,175
0,350
0,700
(USP-Einheiten Heparin/ml Plasma)

FIG.2

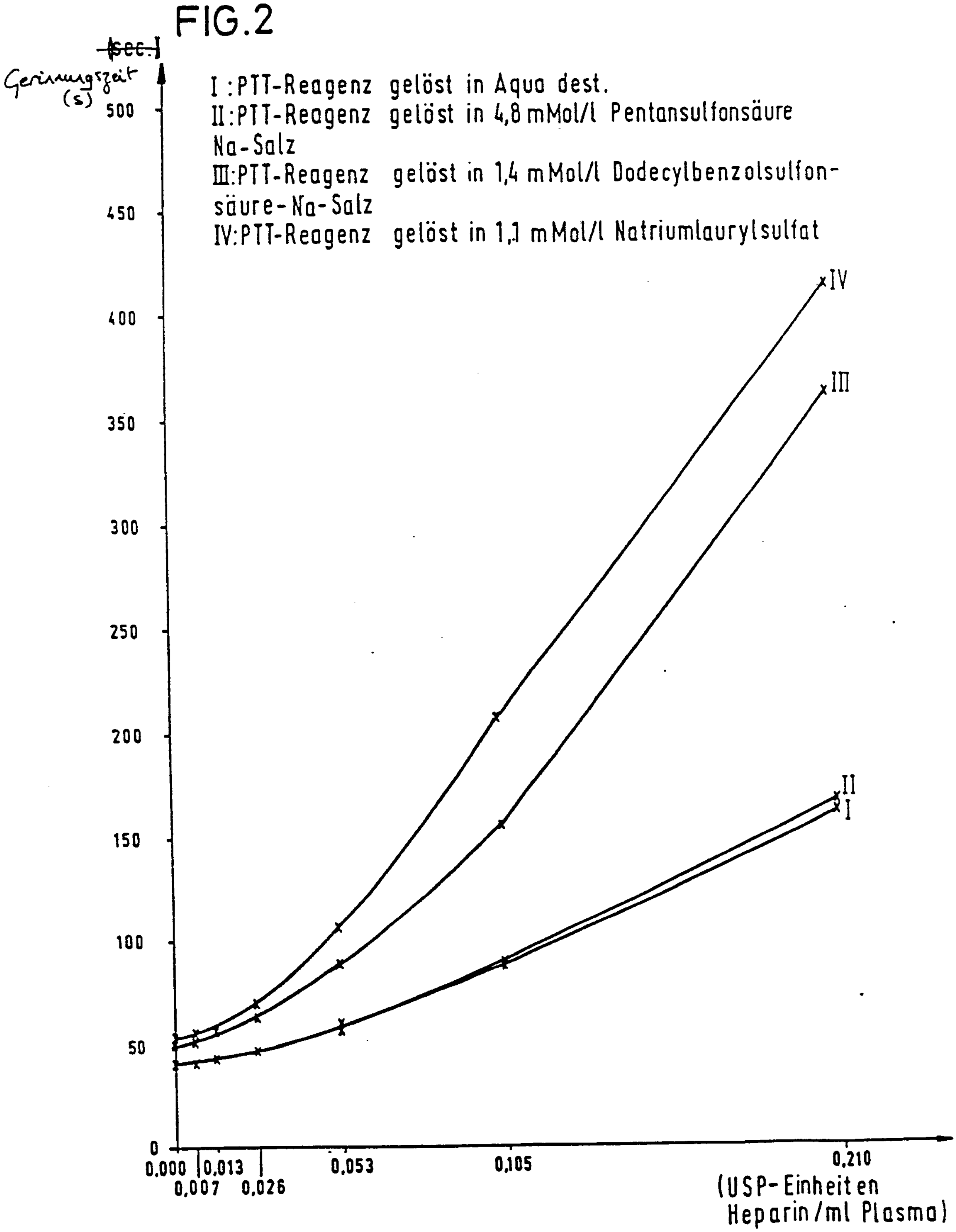
~~sec.~~
Gerinnungszeit (s)
500
450
400
350
300
250
200
150
100
50
0
I :PTT-Reagenz gelöst in Aqua dest.
II:PTT-Reagenz gelöst in 4,8 mMol/l Pentansulfonsäure Na-Salz
III:PTT-Reagenz gelöst in 1,4 mMol/l Dodecylbenzolsulfonsäure-Na-Salz
IV:PTT-Reagenz gelöst in 1,1 mMol/l Natriumlaurylsulfat
IV
III
II
I
0,000
0,007
0,013
0,026
0,053
0,105
0,210
(USP-Einheiten Heparin/ml Plasma)

FIG.3

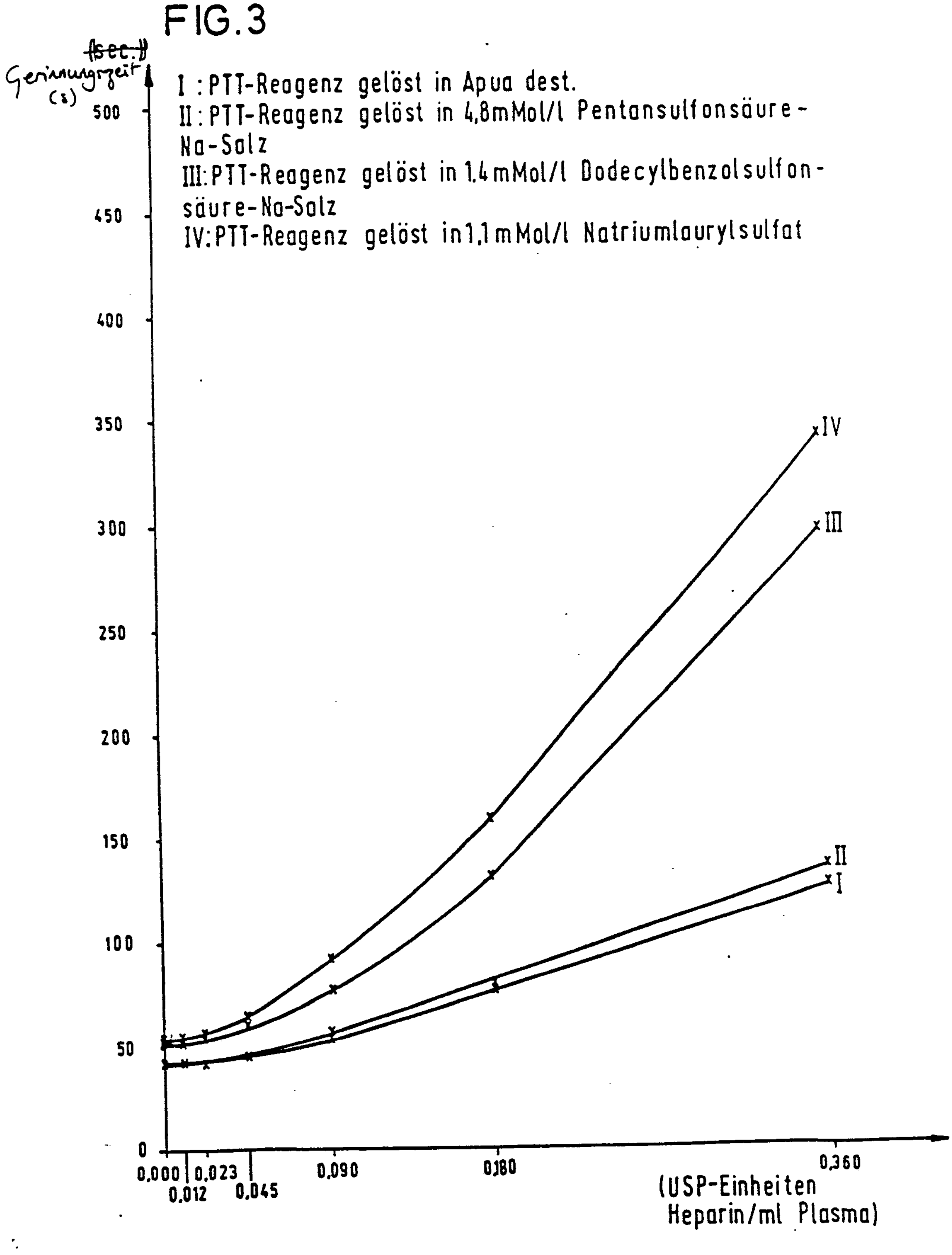
Gerinnungszeit (s)
~~(sec.)~~
I : PTT-Reagenz gelöst in Apua dest.
II: PTT-Reagenz gelöst in 4,8mMol/l Pentansulfonsäure-Na-Salz
III: PTT-Reagenz gelöst in 1.4mMol/l Dodecylbenzolsulfonsäure-Na-Salz
IV: PTT-Reagenz gelöst in 1,1mMol/l Natriumlaurylsulfat
500
450
400
350
300
250
200
150
100
50
0
IV
III
II
I
0.000
0.012
0.023
0.045
0.090
0.180
0.360
(USP-Einheiten Heparin/ml Plasma)